# EUROPEAN PATENT APPLICATION

(11) **EP 4 635 590 A1**
(43) Date of publication of application: **22.10.2025**
(21) Application number: 23903440.8
(22) Date of filing: 08.12.2023
(51) Int. Cl.: B01D 9/02, B01J 4/00, C07C 51/43, C07C 57/07

(54) **METHOD FOR DISCHARGING CRYSTAL GRAIN-CONTAINING SLURRY FROM CONTAINER**

(30) Priority: 16.12.2022 JP 2022201159
(71) Applicant: Nippon Shokubai Co., Ltd., Osaka-shi, Osaka 541-0043 (JP)
(72) Inventor: SANO, Itsumi, Himeji-shi, Hyogo 671-1282 (JP); MUKAE, Masashi, Himeji-shi, Hyogo 671-1282 (JP)
(74) Representative: Schön, Christoph
(86) International application number: PCT/JP2023/044017
(87) International publication number: WO 2024/128153

(57) **Abstract**

Provided is a method for appropriately handling crystal grains. The present invention relates to, for example, a method including: feeding a crystal grain melt-containing liquid into a vessel; and discharging a crystal grain-containing slurry from the vessel, the vessel including therein at least one liquid feed unit including liquid ejection holes for ejecting the melt-containing liquid into the vessel, the liquid feed unit including: a liquid ejection part including the liquid ejection holes in a number of M or more, M being calculated according to a specified requirement; and a liquid feed area for feeding the liquid from outside the vessel into the liquid ejection part, the liquid being ejected from the liquid ejection part into the vessel through the liquid ejection holes in a plurality of different substantially horizontal directions at a liquid ejection velocity of 2 m/s or higher.

## Description

### TECHNICAL FIELD

The present invention relates to methods for discharging a crystal grain-containing slurry from a vessel. Specifically, the present invention relates to a method for discharging a crystal grain-containing slurry from a vessel and a method for producing a compound.

### BACKGROUND ART

Handling of crystal grains is essential in many cases such as in the production and purification of chemical products, and there are many opportunities to handle them on an industrial scale. For example, when easily polymerizable compounds such as (meth)acrylic acid are produced and purified as raw materials for resins and other products, handling of their crystal grains is necessary. In response to this, various excellent methods for handling crystal grains have been studied.

Industrially, many crude compounds are purified through continuous purification. Disclosed is a method for producing acrylic acid including: collecting and crystallization purifying a gas containing acrylic acid obtained by catalytic gas-phase oxidation of a raw material gas; and returning acrylic acid obtained by decomposing a substance obtained by Michael addition of acrylic acid in a residual mother liquor to the collecting step, for example (see, for example, Patent Literature 1). This method enables high-yield production of acrylic acid.

In the purification, a wash column such as a hydraulic wash column (HWC) may be used. For example, Patent Literatures 2 to 4 disclose conventional purification methods using wash columns.

### CITATION LIST

### - Patent Literature

Patent Literature 1: JP 2007-182437 A
Patent Literature 2: EP 1469926 A1
Patent Literature 3: JP 2005-509010 T
Patent Literature 4: JP H11-123302

### SUMMARY OF INVENTION

### - Technical Problem

As discussed above, an excellent method for handling crystal grains has been required. The present invention has been made in view of the above-mentioned problems, and aims to provide a method for appropriately handling crystal grains.

### - Solution to Problem

The present inventors have investigated methods for appropriately handling crystal grains, and have focused on a method for handling crystal grains including: ejecting liquid in a vessel having high washing efficiency such as a hydraulic wash column; and breaking up a crystal bed by the dynamic pressure of the liquid. Then, the present inventors have focused on a method including: feeding a crystal grain melt-containing liquid into a vessel; and discharging a crystal grain-containing slurry from the vessel, the vessel including therein at least one liquid feed unit including liquid ejection holes for ejecting the melt-containing liquid into the vessel, the liquid feed unit including: a liquid ejection part including the liquid ejection holes in a number of M or more, M being calculated according to a specified requirement; and a liquid feed area for feeding the liquid from outside the vessel into the liquid ejection part, the liquid being ejected from the liquid ejection part into the vessel through the liquid ejection holes in a plurality of different substantially horizontal directions at a liquid ejection velocity of 2 m/s or higher. The present inventors found that such a method allows the crystal grains to be handled substantially uniformly, for example, allows the crystal bed to be substantially uniformly broken up by the dynamic pressure of the liquid. Thereby, substantially uniform washing can be achieved and thus products of excellent quality can be obtained. Thereby, the present inventors have arrived at the present invention.

Specifically, an aspect (1) of the present invention relates to a method including:
feeding a crystal grain melt-containing liquid into a vessel; and
discharging a crystal grain-containing slurry from the vessel,
the vessel including therein at least one liquid feed unit including liquid ejection holes for ejecting the melt-containing liquid into the vessel,
the liquid feed unit including:
   a liquid ejection part including the liquid ejection holes in a number of M or more, M being calculated according to the following requirement; and
   a liquid feed area for feeding the liquid from outside the vessel into the liquid ejection part,
   the liquid being ejected from the liquid ejection part into the vessel through the liquid ejection holes in a plurality of different substantially horizontal directions at a liquid ejection velocity of 2 m/s or higher,
   the requirement being as follows:
      Requirement: M is a value obtained by dividing 360 by either of the following values and rounded down to the nearest whole number:
      1) an expansion angle (°) of water ejected in water from a single liquid ejection hole equivalent to any of the liquid ejection holes at the same or almost the same velocity; and
      2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

An aspect (2) of the present invention relates to a method in combination with the aspect (1) of the present invention,
wherein in the at least one liquid feed unit, when viewed from the top to bottom direction, all horizontal ejection difference angles are substantially the same as each other, wherein each horizontal ejection difference angle denotes an angle formed by ejection directions of the liquid ejected from adjacent liquid ejection holes among the liquid ejection holes.

An aspect (3) of the present invention relates to a method in combination with the aspect (1) or (2) of the present invention,
wherein the liquid ejection holes included in the liquid ejection part are provided on a circle having a center in the liquid ejection part when viewed from the top to bottom direction.

An aspect (4) of the present invention relates to a method in any combination with any one of the aspects (1) to (3) of the present invention,
wherein in the at least one liquid feed unit, the liquid ejection holes are provided at two or more different heights when viewed from the horizontal direction.

An aspect (5) of the present invention relates to a method in any combination with any one of the aspects (1) to (4) of the present invention,
wherein the slurry is discharged in at least four directions from the vessel at a linear velocity of 1 m/s or higher.

An aspect (6) of the present invention relates to a method for producing a compound, the method including using a hydraulic wash column,
the hydraulic wash column including therein at least one circulating liquid feed unit including circulating liquid ejection holes for ejecting a crystal grain melt-containing circulating liquid into the hydraulic wash column,
the circulating liquid feed unit including:
   a circulating liquid ejection part including the circulating liquid ejection holes in a number of M or more, M being calculated according to the following requirement; and
   a circulating liquid feed area for feeding a circulating liquid from outside the hydraulic wash column into the circulating liquid ejection part,
   the circulating liquid being ejected from the circulating liquid ejection part into the hydraulic wash column through the circulating liquid ejection holes in a plurality of different substantially horizontal directions at a circulating liquid ejection velocity of 2 m/s or higher, with an amount of the liquid ejected from one circulating liquid ejection hole being 10 kg/h or more,
   the requirement being as follows:
      Requirement: M is a value obtained by dividing 360 by either of the following values and rounded down to the nearest whole number:
      1) an expansion angle (°) of water ejected in water from a single liquid ejection hole equivalent to any of the liquid ejection holes at the same or almost the same velocity; and
      2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

An aspect (7) of the present invention relates to a method in combination with the aspect (6) of the present invention,
wherein, in an inner view of the hydraulic wash column from the top to bottom direction, one circulating liquid feed unit is present per 30 to 90 cm².

An aspect (8) of the present invention relates to a method in combination with the aspect (6) or (7) of the present invention,
wherein a temperature of the circulating liquid is higher than a melting point of the compound by not less than + 1°C.

An aspect (9) of the present invention relates to a method in any combination with any one of the aspects (6) to (8) of the present invention,
wherein the compound is an organic compound having a solid density higher than a liquid density of the organic compound.

An aspect (10) of the present invention relates to a method in any combination with any one of the aspects (6) to (9) of the present invention,
wherein the compound is (meth)acrylic acid.

An aspect (11) of the present invention relates to a method including:
feeding a crystal grain melt-containing liquid into a vessel; and
discharging a crystal grain-containing slurry from the vessel,
the vessel including therein at least one liquid feed unit including liquid ejection holes for ejecting the melt-containing liquid into the vessel,
the liquid feed unit including:
   a liquid ejection part including the liquid ejection holes in a number of M or more, M being calculated according to the following requirement; and
   a liquid feed area for feeding the liquid from outside the vessel into the liquid ejection part,
   the liquid being ejected from the liquid ejection part into the vessel through the liquid ejection holes in a plurality of different substantially horizontal directions at a liquid ejection velocity of 0.3 m/s or higher,
   the requirement being as follows:
      Requirement: M is a value obtained by dividing 360 by either of the following values and rounded down to the nearest whole number:
      1) an expansion angle (°) of water ejected in water from a single liquid ejection hole equivalent to any of the liquid ejection holes at the same or almost the same velocity; and
      2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

An aspect (12) of the present invention relates to a method for producing a compound, the method including using a hydraulic wash column,
the hydraulic wash column including therein at least one circulating liquid feed unit including circulating liquid ejection holes for ejecting a crystal grain melt-containing circulating liquid into the hydraulic wash column,
the circulating liquid feed unit including:
   a circulating liquid ejection part including the circulating liquid ejection holes in a number of M or more, M being calculated according to the following requirement; and
   a circulating liquid feed area for feeding a circulating liquid from outside the hydraulic wash column into the circulating liquid ejection part,
   the circulating liquid being ejected from the circulating liquid ejection part into the hydraulic wash column through the circulating liquid ejection holes in a plurality of different substantially horizontal directions at a circulating liquid ejection velocity of 0.3 m/s or higher, with an amount of the liquid ejected from one circulating liquid ejection hole being 1.5 kg/h or more,
   the requirement being as follows:
      Requirement: M is a value obtained by dividing 360 by either of the following values and rounded down to the nearest whole number:
      1) an expansion angle (°) of water ejected in water from a single liquid ejection hole equivalent to any of the liquid ejection holes at the same or almost the same velocity; and
      2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

Patent Literatures 2 to 4 described above disclose wash columns. Patent Literature 2 discloses ejecting a washing liquid as a jet from a guide device, but does not disclose the number of openings for ejection and the ejection velocity and does not disclose that crystal grains are handled substantially uniformly by adjusting the number of openings and the ejection velocity. Patent Literature 3 discloses a rotating knife disk for removing acrylic acid crystals from the crystal bed. Patent Literature 4 discloses that the crystal bed is broken up using a heat transfer tube in a heating and melting section of the wash column. However, Patent Literatures 3 and 4 do not describe that crystal grains are handled utilizing the dynamic pressure of liquid ejected.

### - Advantageous Effects of Invention

According to the method of the present invention, crystals can be handled substantially uniformly in a vessel such as a hydraulic wash column.

### BRIEF DESCRIPTION OF DRAWINGS

FIG. 1 is an exemplary schematic diagram of a vessel according to a method of the present invention.
FIG. 2 is an exemplary schematic diagram of a liquid feed unit according to the method of the present invention.

### DESCRIPTION OF EMBODIMENTS

The present invention is described in detail below.

It should be noted that combinations of two or more of the preferred features of the present invention described below are also preferred embodiments of the present invention.

First, a method for discharging a crystal grain-containing slurry from a vessel of the present invention is described below. Next, a method for producing a compound of the present invention is described.

### (Method for discharging crystal grain-containing slurry from vessel)

The method for discharging a crystal grain-containing slurry from a vessel of the present invention is, for example, a method including: feeding a crystal grain melt-containing liquid into a vessel; and discharging a crystal grain-containing slurry from the vessel, the vessel including therein at least one liquid feed unit including liquid ejection holes for ejecting the melt-containing liquid into the vessel, the liquid feed unit including: a liquid ejection part including the liquid ejection holes in a number of M or more, M being calculated according to the following requirement; and a liquid feed area for feeding the liquid from outside the vessel into the liquid ejection part, the liquid being ejected from the liquid ejection part into the vessel through the liquid ejection holes in a plurality of different substantially horizontal directions at a liquid ejection velocity of 2 m/s or higher.

The requirement is as follows: Requirement: M is a value obtained by dividing 360 by either of the following values and rounded down to the nearest whole number:
1) an expansion angle (°) of water ejected in water from a single liquid ejection hole equivalent to any of the liquid ejection holes at the same or almost the same velocity; and
2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

In the method of the present invention, the liquid ejection part includes the liquid ejection holes in a number of M or more, M being calculated according to the above requirement, and thus the product of the number of liquid ejection holes (M or more) and the expansion angle of the liquid ejected from any of the liquid ejection holes is 360 or more. Thereby, the liquid can be ejected sufficiently around the liquid feed unit, and the crystal grains can be handled substantially uniformly.

The phrase "single liquid ejection hole equivalent to any of the liquid ejection holes" in determining the expansion angle in 1) and 2) above refers to an ejection hole having substantially the same peripheral thickness, shape, and size as any of the liquid ejection holes of the liquid feed unit used in the method of the present invention. It suffices that the "single liquid ejection hole equivalent to any of the liquid ejection holes" has substantially the same peripheral thickness, shape, and size as described above. It is not necessary to prepare the same unit as the liquid feed unit, nor to use ejection holes provided in the unit. Any unit having the opening may be used.

The phrase "having substantially the same peripheral thickness as any of the liquid ejection holes" means that the difference in the thickness (mm) is within ±50%.

It suffices that the phrase "having substantially the same size as any of the liquid ejection holes" means that the difference in the length, width, or diameter (mm) is within ±0.5 mm.

It suffices that the "same or almost the same velocity" in 1) and 2) above means that the linear velocity (m/s) has the same integer part and the first decimal place as the liquid ejection velocity through any of the liquid ejection holes in the method of the present invention when the value is rounded to one decimal place.

The "water" in 1) above may be tap water or colored industrial water (colored water).

It suffices that the viscosity (cP) and the temperature (°C) of the "liquid having the same or almost the same physical properties" in 2) above have the same integer parts as those of the liquid to be ejected in the method of the present invention when each value is rounded down to the nearest whole number, and that the density (kg/m³) of the "liquid having the same or almost the same physical properties" in 2) above is within ±5% of the density of the liquid to be ejected in the method of the present invention.

The viscosity is measured, for example, using a tuning fork vibration viscometer under conditions of room temperature and atmospheric pressure.

The expansion angle in 1) above may be measured as follows: an opening having the same shape and size as any of the liquid ejection holes of the liquid feed unit is provided on a side of a nozzle or the like that is made of the same material as the periphery of any of the liquid ejection holes of the liquid feed unit and that has the same thickness as the periphery of any of the liquid ejection holes of the liquid feed unit, water is ejected horizontally from the opening until the pressure stabilizes, the valve is then switched, and colored water is ejected horizontally. Then, the distance, width, and behavior of the water ejected from the opening are observed from above and recorded for measurement.

The flow analysis (numerical calculation) in 2) above uses, for example, software manufactured by SIEMENS, but may be performed in any manner.

In both 1) and 2) above, the unit of the expansion angle is degrees (°).

In the liquid feed unit used in the method of the present invention, preferably, the liquid ejection holes are equivalent to each other, and the physical properties and the velocity of the liquid ejected from the liquid ejection holes are the same or almost the same between the liquid ejection holes. The expansion angles of the liquid ejected from the liquid ejection holes are also preferably the same or almost the same as each other. For example, when, in the liquid feed unit, the liquid ejection holes are not equivalent to each other, or the physical properties and the velocity of the liquid ejected from the liquid ejection holes are not the same or almost the same between the liquid ejection holes, the expansion angles of the liquid ejected from the liquid ejection holes may sometimes be different from each other. When, in the liquid feed unit, the liquid ejection holes are not equivalent to each other, and the physical properties and the velocity of the liquid ejected from the liquid ejection holes are not the same or almost the same between the liquid ejection holes, as described above, the expansion angle is measured for each liquid ejection hole using the method described in 1) or 2) above.

In the requirement, when the value obtained by dividing 360 by the value of 1) above (rounded down to the nearest whole number) is different from the value obtained by dividing 360 by the value of 2) above (rounded down to the nearest whole number), the larger value is taken as M. Although the smaller value may be taken as M, the larger value is preferably taken as M.

In the phrase "the liquid being ejected from the liquid ejection part in a plurality of different substantially horizontal directions", the phrase "substantially horizontal directions" may be any direction within ±5° of the horizontal direction, and the phrase "in a plurality of different substantially horizontal directions" means that the circulating liquid is ejected in different directions from a plurality of circulating liquid ejection holes when viewed from the top to bottom direction. The phrase "when viewed from the top to bottom direction" herein means when the vessel is viewed from above (viewed vertically from above with respect to the horizontal plane).

The liquid feed unit includes a liquid feed area for feeding liquid from outside the vessel to the liquid ejection part.

For example, in the liquid feed unit, the liquid feed area may be located below the liquid ejection part including liquid ejection holes and may feed a crystal grain melt-containing liquid to the liquid ejection part from a lower part of the vessel in which crystal grains have accumulated. Alternatively, the liquid feed area may be located above the liquid ejection part including liquid ejection holes and may feed a crystal grain melt-containing liquid to the liquid ejection part from an upper part of the vessel in which crystal grains have accumulated. In particular, preferably, the crystal grain-containing slurry is discharged from the vessel, and a liquid derived from the slurry is returned through a return step and fed to the liquid feed area of the liquid feed unit. In the vessel, the discharged slurry does not need to be recycled. When the density of the crystal grains is greater than the density of the crystal grain melt, the crystal bed moves downward from above, and the crystal grains accumulate in a lower part of the vessel. On the other hand, when the density of the crystal grains is smaller than the density of the crystal grain melt, the crystal bed moves upward from below, and the crystal grains accumulate in an upper part of the vessel.

In the method of the present invention, the number of liquid ejection holes that is M or more is the number per liquid feed unit (one unit).

The liquid ejection holes of the liquid feed unit may be provided at the same or different positions in the top to bottom direction (heights). In other words, the liquid ejection holes may be provided in one row or in two or more rows, i.e., at two or more different heights. When the liquid ejection holes are provided at two or more different heights, the number of the liquid ejection holes refers to the total number thereof.

From the viewpoint of handling the crystals more uniformly, the larger the number of liquid ejection holes, the better. The number of liquid ejection holes is preferably M × 1.1 or more, more preferably M × 1.2 or more, still more preferably M × 1.3 or more, particularly preferably M × 1.5 or more.

There is no upper limit on the number of liquid ejection holes. From the viewpoint of increasing the mechanical strength of the liquid feed unit and of processing precision during fabrication of the liquid feed unit, the number of liquid ejection holes is preferably M × 3 or less, more preferably M × 2.5 or less.

In the present invention, the number of liquid ejection holes may also be specified as an absolute number.

For example, from the viewpoint of handling the crystals more uniformly, the number of liquid ejection holes is preferably 10 or more, more preferably 12 or more, still more preferably 14 or more, particularly preferably 18 or more.

There is no upper limit on the number of liquid ejection holes. The number of liquid ejection holes is preferably 36 or less, more preferably 30 or less.

In the method of the present invention, the number of liquid ejection holes per row in the liquid feed unit is preferably 6 or more, more preferably 7 or more, still more preferably 9 or more.

The number of liquid ejection holes per row is preferably 18 or less, more preferably 15 or less.

In the method of the present invention, it suffices that the liquid ejection velocity is 0.3 m/s or higher from the viewpoint of sufficiently increasing the expansion angle. From the viewpoint of increasing the expansion angle, the liquid ejection velocity is preferably 1 m/s or higher, more preferably 1.5 m/s or higher, still more preferably 2 m/s or higher, particularly preferably 3 m/s or higher.

There is no upper limit on the liquid ejection velocity. The liquid ejection velocity is usually 20 m/s or lower, preferably 15 m/s or lower.

In the method of the present invention, the amount of the liquid ejected from one liquid ejection hole is preferably 1.5 kg/h or more.

The amount of the liquid ejected is more preferably 5 kg/h or more, still more preferably 7.5 kg/h or more, further more preferably 10 kg/h or more, still further more preferably 20 kg/h or more, even still further more preferably 30 kg/h or more.

There is no upper limit on the amount of the liquid ejected. The amount of the liquid ejected is usually 200 kg/h or less, preferably 100 kg/h or less.

In the method of the present invention, the amount of the liquid ejected from one liquid feed unit is preferably 20 kg/h or more.

The amount of the liquid ejected is more preferably 50 kg/h or more, further preferably 100 kg/h or more.

There is no upper limit on the amount of the liquid ejected. The amount of the liquid ejected is usually 2000 kg/h or less, preferably 1000 kg/h or less.

In the method of the present invention, the distance between the center of a liquid ejection hole and the center of an adjacent liquid ejection hole (center-to-center distance) is larger than the diameter of any of the liquid ejection holes, preferably 1.2 times the diameter of any of the liquid ejection holes or larger, more preferably 1.5 times the diameter of any of the liquid ejection holes or larger, from the viewpoint of ensuring the mechanical strength of the liquid feed unit and processing precision during fabrication of the liquid feed unit.

Here, the liquid ejection holes adjacent to each other may be adjacent in the horizontal direction, in the height direction, or in a diagonal direction between the horizontal direction and the height direction.

In the method of the present invention, the distance between adjacent liquid ejection holes is also preferably defined as follows.

### (Distance in horizontal direction)

In the inner circumferential portion of the liquid feed unit, the distance between adjacent ejection holes (length of a portion free from holes) is preferably 0.5 mm or more, more preferably 1.0 mm or more.

### (Distance in vertical direction)

In the outer circumferential portion of the liquid feed unit, the distance between adjacent ejection holes (length of a portion free from holes) is preferably 0.5 mm or more, more preferably 1.0 mm or more.

From the viewpoint of appropriate ejection, the distance between adjacent ejection holes (length of a portion free from holes) is preferably 3 mm or less, more preferably 2 mm or less, still more preferably 1.5 mm or less.

The liquid ejection hole diameter refers to a diameter passing through the center of the liquid ejection hole in the center-to-center direction between the liquid ejection hole and an adjacent liquid ejection hole, and refers to a diameter of the liquid ejection hole when the liquid ejection hole is circular. When the diameters of any two of the liquid ejection holes used to determine the distance are different from each other, the diameter of the larger liquid ejection hole is taken to determine the distance.

The center of the liquid ejection hole is the center of gravity when the liquid ejection hole is assumed to be a solid article of uniform density.

In the method of the present invention, a crystal grain melt-containing liquid is fed into a vessel. In the vessel, after a crystal grain-containing slurry is discharged from the vessel, part of a liquid derived from the slurry (a melt-containing circulating liquid) may be recycled. For example, as described above, the crystal grain-containing slurry is discharged from the vessel, the crystal grains in the slurry are melted, and a liquid derived from the slurry is returned through a return step and fed to the liquid feed area of the liquid feed unit. In the method of the present invention, the liquid derived from the slurry may not be recycled.

In the method of the present invention, the vessel is preferably one in which crystal grains have accumulated in a lower or upper part of the vessel. Examples of the vessel include wash columns such as a hydraulic wash column and a gravity settling wash column, a crystallizer for producing a slurry containing crystal grains of a compound, and an aging tank capable of holding crystals of a compound in a suspended state therein. Of these, a hydraulic wash column is more preferred.

In the method of the present invention, preferably, in at least one liquid feed unit, when viewed from the top to bottom direction, all horizontal ejection difference angles are substantially the same as each other, wherein each horizontal ejection difference angle denotes an angle formed by ejection directions of the liquid ejected from adjacent liquid ejection holes among the liquid ejection holes.

The phrase "all horizontal ejection difference angles are substantially the same as each other" means that the percentage of the difference between each horizontal ejection difference angle and a value of "360°/number of liquid ejection holes" to the value of "360°/number of liquid ejection holes" is within ±25% or the difference between each horizontal ejection difference angle and a value of "360°/number of liquid ejection holes" is within ±10°.

From the viewpoint of handling the crystals more uniformly, the smaller the horizontal ejection difference angle, the better. Each of the horizontal ejection difference angles is preferably 36° or less, more preferably 30° or less.

The horizontal ejection difference angle may be any angle as long as its lower limit exceeds 0°.

In the method of the present invention, in at least one liquid feed unit, when viewed from the top to bottom direction, the difference between the maximum horizontal ejection difference angle and the minimum horizontal ejection difference angle is preferably 10° or less, more preferably 5° or less, wherein each horizontal ejection difference angle denotes an angle formed by ejection directions of the liquid ejected from adjacent liquid ejection holes among the liquid ejection holes.

In the method of the present invention, preferably, the liquid ejection holes included in the liquid ejection part are provided on a circle having a center in the liquid ejection part when viewed from the top to bottom direction.

The phrase "having a center in the liquid ejection part" refers to the center of gravity of the liquid ejection part when having, for example, a cylindrical shape including a plurality of liquid ejection holes. The center may also be a portion free from structural member of the liquid ejection part (the interior of the cylinder through which the liquid passes).

A state where the liquid ejection holes are provided in two or more rows at two or more different heights and are all provided on a circle having a center in the liquid ejection part when viewed from the top to bottom direction is also encompassed in the feature that "the liquid ejection holes included in the liquid ejection part are provided on a circle having a center in the liquid ejection part when viewed from the top to bottom direction".

In the method of the present invention, preferably, when the vessel includes liquid feed units, in 50% or more of the liquid feed units, all the horizontal ejection difference angles are substantially the same as each other. More preferably, in substantially all the liquid feed units in the vessel, all the horizontal ejection difference angles are substantially the same as each other.

In the method of the present invention, preferably, in at least one liquid feed unit, the liquid ejection holes are provided at two or more different heights when viewed from the horizontal direction.

Providing the liquid ejection holes in two or more rows ensures a more sufficient distance between the liquid ejection holes (shortest distance), particularly in the horizontal direction, and allows the liquid feed unit to have better mechanical strength.

There is no upper limit on the number of rows. The number of rows is preferably, for example, 5 or less.

In the method of the present invention, preferably, the slurry is discharged in at least four directions from the vessel at a linear velocity of 1 m/s or higher.

The linear velocity is more preferably 1.2 m/s or higher.

There is no upper limit on the linear velocity. The linear velocity is preferably 10 m/s or lower.

There is no upper limit on the number of directions in which the slurry is discharged. The number of directions is preferably 10 or less.

In other words, the slurry is preferably discharged from at least four slurry discharge ports provided at the bottom of the vessel. The number of slurry discharge ports provided at the bottom of the vessel is preferably 10 or less.

Preferably, when the directions in which the slurry is discharged are viewed from the top to bottom direction, all horizontal discharge difference angles are substantially the same as each other, wherein each horizontal discharge difference angle denotes an angle formed by discharge directions of the slurry discharged from adjacent slurry discharge ports among the slurry discharge ports provided in the vessel.

The phrase "all horizontal discharge difference angles are substantially the same as each other" means that the difference between the maximum horizontal discharge difference angle and the minimum horizontal discharge difference angle is within ±20% of the minimum horizontal discharge difference angle.

In the method of the present invention, the amount of the slurry discharged from the vessel is preferably 1000 kg/h or more, more preferably 3000 kg/h or more, still more preferably 10000 kg/h or more.

There is no upper limit on the amount of the slurry discharged from the vessel. The amount is usually 500000 kg/h or less, preferably 300000 kg/h or less.

In the method of the present invention, the slurry discharge ports are preferably disposed on a circle having a center in the vessel when viewed from the top to bottom direction.

The center in the vessel may be, for example, the center of gravity of the vessel.

The vessel used in the method of the present invention may have any dimensions. Preferably, the inner diameter is 30 to 2000 mm, for example. The height is preferably 1000 to 15000 mm. Use of the method of the present invention allows crystal grains to be handled substantially uniformly even in an industrial-scale vessel.

The liquid feed unit in the vessel may have any dimensions. Preferably, the inner diameter is 5 to 30 mm, for example. The height of the unit is preferably 10 to 300 mm.

The liquid ejection part and the liquid feed area of the liquid feed unit may differ in inner diameter and height.

Preferably, in an inner view of the vessel from the top to bottom direction, one (one unit) liquid feed unit is present per 30 to 90 cm². In other words, preferably, the liquid feed units are disposed at a number density of 30 to 90 cm²/unit evenly inside the vessel or disposed to correspond to the members included in the vessel.

The number of the liquid feed units is more preferably one per 30 to 70 cm², more preferably one per 30 to 50 cm².

In the method of the present invention, the thickness of the liquid ejection part of the liquid feed unit (the peripheral thickness of the liquid ejection holes) is preferably 0.1 to 20 mm, more preferably 0.5 to 15 mm, still more preferably 1 to 10 mm.

The material of the liquid ejection part of the liquid feed unit (the material of the periphery of the liquid ejection holes) may be any material and may be metal, resin, or the like.

The thickness and material of the liquid feed area are the same or almost the same as those of the liquid ejection part described above.

In the method of the present invention, a mechanism for discharging crystals from the crystal bed formed in the vessel, such as a rotor blade or a scraper, may be used in combination with the liquid feed unit. Preferably, such a mechanism is not used.

The method of the present invention can be applied to a production method of the present invention, which is described later. Also, the production method of the present invention, which is described later, can be applied to the method of the present invention.

### (Method of producing compound)

The present invention also relates to, for example, a method for producing a compound, the method including using a hydraulic wash column, the hydraulic wash column including therein at least one circulating liquid feed unit including circulating liquid ejection holes for ejecting a crystal grain melt-containing circulating liquid into the hydraulic wash column, the circulating liquid feed unit including: a circulating liquid ejection part including the circulating liquid ejection holes in a number of M or more, M being calculated according to the following requirement; and a circulating liquid feed area for feeding a circulating liquid from outside the hydraulic wash column into the circulating liquid ejection part, the circulating liquid being ejected from the circulating liquid ejection part into the hydraulic wash column through the circulating liquid ejection holes in a plurality of different substantially horizontal directions at a circulating liquid ejection velocity of 2 m/s or higher, with an amount of the liquid ejected from one circulating liquid ejection hole being 10 kg/h or more.

The requirement is as follows: Requirement: M is a value obtained by dividing 360 by either of the following values and rounded down to the nearest whole number:
1) an expansion angle (°) of water ejected in water from a single liquid ejection hole equivalent to any of the liquid ejection holes at the same or almost the same velocity; and
2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

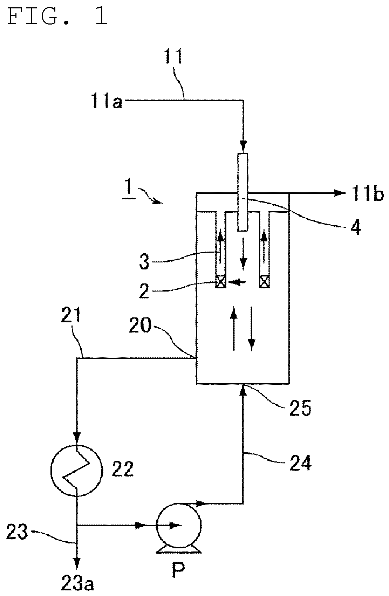

In the hydraulic wash column, usually, a slurry containing crystals of a compound is fed into the hydraulic wash column, and then a crystal bed formed in an upper or lower part of the hydraulic wash column is broken up, and a crystal-containing slurry is discharged and circulated as a circulating slurry or a circulating liquid. (For example, as shown in FIG. 1, a crystal-containing slurry 11a is fed into a hydraulic wash column 1 through a slurry feed line 11 and a slurry feed pipe 4. The crystals deposit in the lower part of the hydraulic wash column 1 to form a crystal bed. Although not shown, a circulating liquid feed unit is disposed in the hydraulic wash column 1 and ejects a circulating liquid to break up the crystal grains substantially uniformly. Then, the crystals are discharged from the bottom of the hydraulic wash column 1 together with the circulating liquid through a slurry discharge port 20 as a crystal-containing circulating slurry. The crystal-containing circulating slurry passes through a slurry circulation line 21 that connects the slurry discharge port 20 and a melting unit 22, and the crystals in the circulating slurry are melted in the melting unit 22. A portion of the circulating liquid containing a melt obtained by melting the crystals in the melting unit 22 is returned into the hydraulic wash column 1 through a return line 24 that connects the melting unit 22 and a return port 25. At least a portion of the returned circulating liquid is fed to the circulating liquid feed area of the circulating liquid feed unit. The rest of the returned circulating liquid may serve as a washing liquid for washing the crystals. The rest of the circulating liquid passes through a product discharge line 23 branched from the return line 24 and is discharged from a purification apparatus as a product 23a.)

The following describes, firstly, the feed of the circulating liquid from the circulating liquid feed unit to the hydraulic wash column in order to break up the crystal bed substantially uniformly when the crystal-containing circulating slurry is discharged, and secondly, other steps including a step of feeding the slurry containing crystals of a compound to the hydraulic wash column, a melting step, and a return step. In a continuous purification step, a step of feeding the slurry containing crystals of a compound to the hydraulic wash column, a step of feeding the circulating liquid from the circulating liquid feed unit to the hydraulic wash column, a step of discharging the crystal-containing circulating slurry, a melting step, and a return step are performed in this order on a target for purification. In the entire purification apparatus, the steps are simultaneously performed.

Herein, the term "compound" refers to a compound to be obtained by the production method of the present invention, and does not refer to raw materials, by-products, or solvents in the production method of the present invention. The term "compound" may also be referred to as a "target compound" or a "target object". Herein, the term "impurities" refers to components other than the "compound", such as raw materials, by-products, and solvents.

### <Feed of circulating liquid from circulating liquid feed unit to hydraulic wash column>

The circulating liquid feed unit includes a circulating liquid ejection part including M or more circulating liquid ejection holes, M being calculated according to the following requirement:
Requirement: M is a value obtained by dividing 360 by either of the following values and rounded down to the nearest whole number:
1) an expansion angle (°) of water ejected in water from a single liquid ejection hole equivalent to any of the liquid ejection holes at the same or almost the same velocity; and
2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

The production method of the present invention is the same as the method of the present invention including discharging a crystal grain-containing slurry from a vessel, except that the amount of the liquid ejected from one circulating liquid ejection hole is specified, the vessel is specified as a hydraulic wash column, and the melt-containing liquid is specified as a melt-containing circulating liquid.

For example, the "single liquid ejection hole equivalent to any of the liquid ejection holes" in the production method of the present invention is the same as the "single liquid ejection hole equivalent to any of the liquid ejection holes" in the method of the present invention including discharging a crystal grain-containing slurry from a vessel. Furthermore, the "same or almost the same velocity" and the "liquid having the same or almost the same physical properties" in the production method of the present invention are respectively the same as the "same or almost the same velocity" and the "liquid having the same or almost the same physical properties" in the method of the present invention including discharging a crystal grain-containing slurry from a vessel.

Other terms are also the same as those described above unless expressly specified.

Since the amount of the liquid ejected from one circulating liquid ejection hole is specified, the production method of the present invention can break up substantially uniformly the crystal bed formed in a lower or upper part of the hydraulic wash column.

The circulating liquid feed unit includes a circulating liquid feed area for feeding a circulating liquid from outside the hydraulic wash column into the circulating liquid ejection part.

In the circulating liquid feed unit, for example, the circulating liquid feed area may be located below the circulating liquid ejection part including circulating liquid ejection holes and may feed a crystal grain melt-containing circulating liquid to the circulating liquid ejection part from a lower part of the hydraulic wash column in which crystal grains have accumulated. Alternatively, the circulating liquid feed area may be located above the circulating liquid ejection part including circulating liquid ejection holes and may feed a crystal grain melt-containing circulating liquid to the circulating liquid ejection part from an upper part of the hydraulic wash column in which the crystal grains have accumulated. In the production method of the present invention, for example, preferably, the crystal grain-containing slurry is discharged from the hydraulic wash column, the circulating liquid containing the melt obtained by melting the crystal grains in the slurry is returned through a return step, fed to the circulating liquid feed area of the circulating liquid feed unit, and fed from the circulating liquid feed area to the circulating liquid ejection part.

Here, the circulating liquid feed area is connected to the return port of the hydraulic wash column at the bottom of the circulating liquid feed unit.

In the production method of the present invention, the circulating liquid ejection velocity may be 0.3 m/s or higher. From the viewpoint of increasing the expansion angle, the circulating liquid ejection velocity is preferably 1 m/s or higher, more preferably 1.5 m/s or higher, still more preferably 2 m/s or higher, particularly preferably 3 m/s or higher.

There is no upper limit on the circulating liquid ejection velocity. The ejection velocity is usually 20 m/s or lower, preferably 15 m/s or lower.

In the production method of the present invention, the amount of the liquid ejected from one circulating liquid ejection hole may be 1.5 kg/h or more. With such an amount, the crystal bed can be broken up substantially uniformly.

The amount of the liquid ejected is preferably 5 kg/h or more, more preferably 7.5 kg/h or more, still more preferably 10 kg/h or more, further more preferably 20 kg/h or more, particularly preferably 30 kg/h or more.

There is no upper limit on the amount of the liquid ejected. The amount is usually 200 kg/h or less, preferably 100 kg/h or less.

In the production method of the present invention, the amount of the liquid ejected from one circulating liquid feed unit is preferably 20 kg/h or more.

The amount of the liquid ejected is preferably 50 kg/h or more, more preferably 100 kg/h or more.

There is no upper limit on the amount of the liquid ejected. The amount of the liquid ejected is usually 2000 kg/h or less, preferably 1000 kg/h or less.

In the production method of the present invention, when a hydraulic wash column with a larger diameter, which includes a greater number of circulating liquid feed units, is used, an amount of the liquid ejected can be increased. For this reason, the production method of the present invention is preferably applied to production of bulk chemicals (mass-produced chemicals) such as (meth)acrylic acid.

In the production method of the present invention, preferably, in an inner view of the hydraulic wash column from the top to bottom direction, one (one unit) circulating liquid feed unit is present per 30 to 90 cm². In other words, preferably, the circulating liquid feed units are disposed at a number density of 30 to 90 cm²/unit inside the hydraulic wash column to correspond to the hydraulic wash column or the members (e.g., filters) included in the hydraulic wash column.

Thereby, the crystal grains can be handled more uniformly, resulting in more uniform washing.

The number of the circulating liquid feed units is more preferably one per 30 to 70 cm², more preferably one per 30 to 50 cm².

In the production method of the present invention, preferably, the temperature of the circulating liquid is higher than the melting point of the compound by not less than + 1°C.

There is no upper limit on the temperature of the circulating liquid. The temperature is usually preferably higher than the melting point of the compound by not more than + 20°C, more preferably higher than the melting point of the compound by not more than + 15°C.

For example, when the compound is acrylic acid, the temperature of the circulating liquid is preferably 14°C or higher.

In this case, the temperature of the circulating liquid is usually 33°C or lower, preferably 28°C or lower.

In the production method of the present invention, preferably, the compound is an organic compound having a solid density higher than the liquid density of the organic compound.

When the compound is an organic compound having a solid density higher than the liquid density of the organic compound, the crystal bed moves downward from above, and the crystal grains accumulate in a lower part.

In the production method of the present invention, the melting point of the compound is preferably 0°C to 80°C, more preferably 1°C to 50°C, still more preferably 3°C to 40°C, particularly preferably 5°C to 20°C.

The compound having a melting point within the above range is preferably an easily polymerizable compound having a reactive double bond.

In particular, in the production method of the present invention, the compound is more preferably an unsaturated carboxylic acid, still more preferably (meth)acrylic acid, particularly preferably acrylic acid. Herein, the term "(meth)acrylic acid" refers to acrylic acid and/or methacrylic acid.

Even when the compound is an easily polymerizable compound having a reactive double bond, such as (meth)acrylic acid, the production method of the present invention can be preferably applied thereto.

In the production method of the present invention, the outer wall surface of the hydraulic wash column is preferably heated.

Heating the outer wall surface of the hydraulic wash column can prevent freezing, thereby leading to stable production of the compound.

The outer wall surface of the hydraulic wash column is preferably heated by a heating medium.

The heating medium may be any liquid or gas, and examples include water, antifreeze, a methanol water mixture (an aqueous methanol solution), and gas. The heating medium may be appropriately selected in consideration of the freezing point of the compound to be purified, for example.

The flow rate of the heating medium may be appropriately selected so that the difference between the inlet temperature and the outlet temperature of the heating medium is less than 5°C, preferably less than 3°C, more preferably less than 1°C.

In the production method of the present invention, the outer wall surface of the hydraulic wash column is preferably heated by a heating medium having a temperature higher than the melting point of the compound by at least 3°C.

As described above, the temperature of the heating medium is preferably higher than the melting point of the compound by not less than 3°C, more preferably by not less than 5°C, still more preferably by not less than 7°C.

The temperature of the heating medium is preferably higher than the melting point of the compound by not more than 30°C, more preferably by not more than 20°C. In other words, the temperature of the heating medium is usually higher than the melting point of the compound, and the difference therebetween is preferably not more than 30°C, more preferably not more than 20°C. The melting point of the compound refers to the melting point of a target compound and is preferably 0°C to 80°C, more preferably 1°C to 50°C, still more preferably 3°C to 40°C, particularly preferably 5°C to 20°C.

The heating may be performed by heating part of the hydraulic wash column with a heating medium or the like. Preferably, substantially the entire hydraulic wash column is heated (jacket heating).

In the jacket heating, when the heating medium is liquid, the heating medium is preferably fed from a lower part of the jacket. In this case, the temperature of the heating medium is preferably the same as the inlet temperature.

The heating medium may be fed from an upper part of the jacket. In this case, the temperature of the heating medium preferably refers to the outlet temperature.

During operation, the inside of the hydraulic wash column is basically under pressure. (The pressure is preferably within the range of 0.05 to 1.0 MPa.)

### <Step of feeding slurry containing crystals of compound to hydraulic wash column>

The production method of the present invention preferably includes a step of feeding a slurry containing crystals of a compound to a hydraulic wash column. The crystal-containing slurry is a suspension containing crystals of a compound and a mother liquor. In other words, the liquid portion of the slurry containing crystals of a compound to be fed to the hydraulic wash column is the mother liquor. The crystal-containing slurry can be obtained by forming crystals in a compound-containing solution (e.g., an aqueous (meth)acrylic acid solution or a crude (meth)acrylic acid solution) as described later. The compound-containing solution may be prepared in-house or procured from outside sources. The compound-containing solution encompasses a crude compound.

The mass percentage of the crystals in the crystal-containing slurry to be fed to the hydraulic wash column is preferably 1% by mass or more, more preferably 3% by mass or more, still more preferably 5% by mass or more.

The mass percentage of the crystals is preferably 50% by mass or less, more preferably 40% by mass or less, still more preferably 30% by mass or less, particularly preferably 20% by mass or less.

Herein, the phrase "crystal-containing slurry to be fed to the hydraulic wash column" refers to the crystal-containing slurry immediately before being fed to the hydraulic wash column.

Preferably, in the crystal-containing slurry to be fed to the hydraulic wash column, the mother liquor contains the compound. Examples of the mother liquor include the above-described compound and an aqueous solution of the compound. The mother liquor usually contains impurities other than the compound and water.

In the method for producing a compound of the present invention, the purity (mass percentage) of the compound in the mother liquor in the crystal-containing slurry to be fed to the hydraulic wash column is preferably 99% by mass or less. Thereby, the effect of the present invention can be significantly achieved.

More preferably, the mass percentage of the compound in the mother liquor is 98% by mass or less.

The mass percentage of the compound in the mother liquor is preferably 85% by mass or more, more preferably 88% by mass or more, still more preferably 90% by mass or more.

The mass percentage of water in the mother liquor is preferably 0.1% by mass or more, more preferably 0.5% by mass or more, still more preferably 1% by mass or more.

The mass percentage of water in the mother liquor is preferably 8% by mass or less, more preferably 6% by mass or less, still more preferably 4% by mass or less.

The mass percentage of impurities other than the compound and water in the mother liquor is preferably 0.1% by mass or more, more preferably 0.4% by mass or more, still more preferably 0.8% by mass or more, in order to make the effect of the present invention significant.

The mass percentage of impurities other than the compound and water in the mother liquor is preferably 8% by mass or less, more preferably 6% by mass or less, still more preferably 4% by mass or less.

When the compound is (meth)acrylic acid, the impurities other than the compound and water may include acetic acid and furfural, for example.

In this case, the mass percentage of acetic acid in the mother liquor is preferably 0.1% by mass or more, more preferably 0.3% by mass or more, still more preferably 0.7% by mass or more, in order to make the effect of the present invention significant.

The mass percentage of acetic acid in the mother liquor is preferably 8% by mass or less, more preferably 6% by mass or less, still more preferably 4% by mass or less.

When the compound is (meth)acrylic acid, the mass percentage of furfural in the mother liquor is more preferably 0.01% by mass or more, more preferably 0.05% by mass or more, still more preferably 0.1% by mass or more, in order to make the effect of the present invention significant.

The mass percentage of furfural in the mother liquor is preferably 2% by mass or less, more preferably 1% by mass or less, still more preferably 0.5% by mass or less.

In the feeding step, the crystal-containing slurry may be fed at any feed rate. For example, the feed rate is 0.2 × 10³ to 4.0 × 10⁵ kg/h in an industrial-scale hydraulic wash column.

In the feeding step, the feed temperature of the crystal-containing slurry can be appropriately selected according to the melting point of the compound or the like. For example, the feed temperature may be appropriately adjusted within the range of 0°C to 80°C.

For example, when the compound is (meth)acrylic acid, the feed temperature of the crystal-containing slurry is preferably 5°C to 13°C, more preferably 6°C to 12°C.

The feed temperature of the crystal-containing slurry is the temperature of the mother liquor in the crystal-containing slurry immediately before being fed to the hydraulic wash column.

### <Melting step>

The production method of the present invention preferably includes discharging the crystal grain-containing circulating slurry from the hydraulic wash column and melting the crystal grains contained in the circulating slurry discharged.

The crystal grains originate from the crystal bed formed in a lower part or upper part of the hydraulic wash column. The crystal grains can be suitably discharged by breaking up the crystal bed using the circulating liquid feed unit described above.

The crystals are usually discharged together with the circulating liquid, i.e., as a crystal-containing circulating slurry. This circulating slurry is subjected to the melting step.

The mass percentage of the crystals in the crystal-containing circulating slurry discharged from the hydraulic wash column is preferably 0.5% by mass or more, more preferably 1% by mass or more, still more preferably 3% by mass or more, particularly preferably 5% by mass or more.

The mass percentage of the crystals is preferably 40% by mass or less, more preferably 30% by mass or less, still more preferably 20% by mass or less, particularly preferably 10% by mass or less.

Herein, the crystal-containing circulating slurry and crystals discharged from the hydraulic wash column respectively refer to the crystal-containing circulating slurry and crystals immediately after being discharged from the hydraulic wash column, and, for example, refers to the crystal-containing circulating slurry and crystals in the slurry circulation line that connect the slurry discharge port and the melting unit.

The crystal-containing circulating slurry is discharged from the hydraulic wash column at a discharge rate of 2 × 10³ to 5 × 10⁵ kg/h in an industrial-scale hydraulic wash column, for example, but is not limited thereto.

As described above, the hydraulic wash column may be provided with a plurality of slurry discharge ports, and a plurality of slurry circulation lines may be present.

The discharged crystals can be melted using a heating unit. Examples of the heating unit include those having a structure that efficiently transfers heat to the crystal-containing slurry, such as a vertical multitubular heat exchanger, a horizontal multitubular heat exchanger, a double pipe heat exchanger, a spiral heat exchanger, a plate heat exchanger, or an electric heater. Preferably, the heating unit is provided in a melt loop, and the circulating slurry (circulating liquid after the melting step) circulates in a forced circulation system driven by a pump provided in the melt loop.

The heating temperature in the melting step may be appropriately selected according to the melting point of the compound, and can be adjusted within the range of 10°C to 100°C, for example.

For example, when the compound is (meth)acrylic acid, the heating temperature in the melting step is preferably 15°C or higher. The heating temperature is preferably 50°C or lower, more preferably 40°C or lower.

When the heating is performed by feeding a heating medium to the melting unit, the heating temperature in the melting step is the temperature of the heating medium.

The temperature of the melt-containing circulating liquid at the outlet of the melting step (the melting unit) is preferably set to a temperature 1°C to 20°C higher, more preferably 1°C to 15°C higher, than the melting point of the circulating liquid containing the melt obtained in the melting step (e.g., the circulating liquid containing the melt that is obtained by melting the crystals in the slurry that has passed through the heat exchanger or the like).

The melting time in the melting step may be appropriately selected so that the crystals are sufficiently melted.

### <Return step>

The production method of the present invention preferably includes a step of returning a portion of the circulating liquid containing the melt obtained in the melting step to the hydraulic wash column. The melt is contained in the circulating liquid and thus cannot be separated as a melt.

The circulating liquid contains the melt obtained in the melting step. In other words, the crystals in the discharged circulating slurry are melted to be a melt, so that the suspended circulating slurry becomes a non-suspended circulating liquid.

The circulating liquid circulates as follows: a crystal-containing circulating slurry is discharged from the hydraulic wash column; and a portion thereof is returned to the hydraulic wash column as a circulating liquid containing a melt obtained in a melting step and passes through the hydraulic wash column for circulation. In other words, the circulating liquid flows through a circulation path that passes through the hydraulic wash column. Herein, the liquid component in the circulating slurry flowing through the circulation path is also referred to as the circulating liquid.

The melt obtained in the melting step refers to a liquid obtained by melting the crystals contained in the circulating slurry discharged from the hydraulic wash column in the melting step. The melt does not encompass those derived from the circulating liquid (liquid component) in the circulating slurry.

Here, the circulating slurry is a suspension containing the crystals of a compound and the circulating liquid, and flows through the circulation path.

The circulation path is a circulation path that passes through the hydraulic wash column. A specific example thereof is a circulation path including: slurry circulation lines that connect the slurry discharge ports of the hydraulic wash column and the melting unit; and a return line that connects the melting unit and the return port of the hydraulic wash column. The circulating slurry or the melt-containing circulating liquid circulates through the circulation path. Herein, the circulation path is also referred to as a melt loop.

In the circulation path, a part through which the circulating slurry flows is a part between a point where the crystals in the hydraulic wash column are introduced into the circulating liquid to provide a circulating slurry and a point where the crystals in the circulating slurry are melted. For example, in the melt loop, the circulating liquid returned through the return port 25 provided at the bottom of the hydraulic wash column is ejected from the circulating liquid ejection part of the circulating liquid feed unit and fed into the hydraulic wash column, the circulating liquid mixes with the crystals in the hydraulic wash column to provide a circulating slurry, and the circulating slurry flows through a path (the slurry circulation line 21) between the slurry discharge port 20 and the melting unit 22. A portion of the returned circulating liquid may not be introduced into the circulating liquid feed unit and may be serve as a washing liquid flowing countercurrent to the moving direction of the crystals (bed). The washing liquid constitutes a portion of the circulating liquid returned to the hydraulic wash column and refers to liquid fed to the circulating liquid feed unit without recirculating through the circulation path after returning to the hydraulic wash column, and, for example, flowing countercurrent to the moving direction of the crystals (preferably upward) through gaps between the crystals of the crystal bed in the hydraulic wash column. Thereby, the crystals in the hydraulic wash column are washed.

A product is extracted from the circulating liquid flowing through the circulation path and removed. Meanwhile, the crystals are discharged from the hydraulic wash column and introduced into the circulating liquid flowing through the circulation path. The amount removed from the circulation path and the amount introduced into the circulation path are balanced during the continuous operation. The sum of the amount of the product removed and the amount of the washing liquid is equal to the amount of the crystals discharged from the hydraulic wash column, i.e., the amount of the melt obtained in the melting step.

In the return step, as described above, a portion of the circulating liquid containing the melt obtained in the melting step is returned to the hydraulic wash column. When a portion of the circulating liquid returned to the hydraulic wash column serves as a washing liquid, the washing liquid is preferably returned such that it flows countercurrent to the moving direction of the crystals (bed). The direction may be appropriately determined depending on the specific gravities of the washing liquid and the crystals. For example, when the specific gravity of the crystals is greater than the specific gravity of the mother liquor, the circulating liquid is preferably returned such that it flows upward. The term "upward" preferably refers to a substantially vertically upward direction with respect to the horizontal plane. Thus, the crystals can be washed efficiently.

In an industrial-scale hydraulic wash column, the product removing rate at which the product is removed in accordance with the returning step is 5 kg/h to 4.0 × 10⁴ kg/h.

In the production method of the present invention, a mechanism for controlling the amount of the circulating liquid to be returned (control mechanism) can be used as appropriate. An example of the control mechanism is a valve installed in a line in a return mechanism (branched line).

In the production method of the present invention, use of the control mechanism for controlling the amount of the circulating liquid to be returned enables adjustment of the amount of the circulating liquid to be returned and optionally enables separation of impurities with excellent efficiency. Thereby, a product can be efficiently obtained.

The control mechanism may directly or indirectly control the amount of the circulating liquid to be returned.

When the control mechanism directly controls the amount of the circulating liquid to be returned, the control mechanism may be a valve (not shown) installed in the return line 24 shown in FIG 1, for example.

When the control mechanism indirectly controls the amount of the circulating liquid to be returned, the control mechanism may be a valve (not shown) installed in the product discharge line 23 that is connected to a product discharge port (not shown), for example. The amount of the circulating liquid to be returned through the return line 24 can be controlled resultantly by adjusting the valve installed in the product discharge line 23.

A valve may be installed in both the product discharge line 23 and the return line 24.

For example, the valves can be controlled according to the flow rates in the product discharge line 23 and the return line 24. Furthermore, the valves may be controlled according to the temperature in the hydraulic wash column measured with a multi-point thermometer attached in the hydraulic wash column.

### <Mother liquor discharging step>

The production method of the present invention preferably further includes a step of discharging a mother liquor, the step including filtering the crystal-containing slurry in the hydraulic wash column with a filter and discharging the mother liquor through a pipe connected to the filter. The discharged mother liquor can be recycled and used. For example, recycling of the discharged mother liquor at least as a portion of the crystal-containing slurry to be fed to the hydraulic wash column can provide a compound with further improved quality.

When the specific gravity of the crystals is higher than that of the mother liquor, the mother liquor in the slurry fed in the feeding step flows downward from above, encounters the washing liquid flowing upward from below to be pushed back, and discharged through the filter.

The mother liquor discharged in the mother liquor discharging step usually contains the compound. Examples of the mother liquor include a melt of the compound and an aqueous solution of the compound. The mother liquor usually contains impurities other than the compound and water.

The mother liquor discharged in the mother liquor discharging step refers to the mother liquor immediately after passing through the filter in the mother liquor discharging step.

The mother liquor discharging step can be appropriately performed using a pump or the like.

In a preferred embodiment of the production method of the present invention, when the filter and a pipe connected to the filter are used, the circulating liquid feed unit is disposed below the filter.

### <Step of preparing crystal-containing slurry>

The production method of the present invention preferably further includes a step of preparing a slurry containing crystals of a compound from a compound-containing solution.

The compound-containing solution can be prepared by collecting the gas of a compound, which is a reaction product obtained by a reactor, in an absorption column, for example. The compound-containing solution encompasses a crude compound obtained by purifying the collected compound. The compound-containing solution is not limited to one synthesized in-house, and may be one procured from outside sources.

The compound-containing solution is cooled, for example, and thereby the slurry containing crystals of a compound can be obtained.

The compound-containing solution contains impurities other than the compound and water.

In the production method of the present invention, the compound-containing solution is preferably an aqueous (meth)acrylic acid solution or a crude (meth)acrylic acid solution.

The aqueous (meth)acrylic acid solution refers to a solution in which (meth)acrylic acid is dissolved in water. The crude (meth)acrylic acid solution is a solution composed of (meth)acrylic acid and containing impurities such as by-products produced during the production of (meth)acrylic acid.

Examples of the impurities include acids such as propionic acid, acetic acid, maleic acid, benzoic acid, and acrylic acid dimers; aldehydes such as acrolein, furfural, formaldehyde, and glyoxal; methyl isobutyl ketone; toluene; protoanemonin; and acetone.

The production method of the present invention can sufficiently remove the impurities in the compound-containing solution.

### <Step of preparing compound-containing solution>

In the production method of the present invention, the production method preferably further includes a step of preparing the compound-containing solution from a raw material.

The step of preparing the compound-containing solution may be any step that can provide the compound-containing solution. When the compound is (meth)acrylic acid, the step can be suitably carried out by synthesizing acrylic acid, collecting the acrylic acid, and the like, as described in JP 2007-182437 A (Patent Literature 1), for example.

In the method for producing a compound of the present invention, the raw material is preferably at least one selected from the group consisting of propane, propylene, acrolein, isobutene, methacrolein, acetic acid, lactic acid, isopropanol, 1,3-propanediol, glycerol, and 3-hydroxypropionic acid. The (meth)acrylic acid and/or the raw material may also be bio-based (meth)acrylic acids derived from renewable raw materials.

In the step of preparing the compound-containing solution, impurities such as by-products basically generate. For example, when the compound is (meth)acrylic acid, the impurities generated include water; acids such as propionic acid, acetic acid, maleic acid, benzoic acid, and acrylic acid dimers; aldehydes such as acrolein, furfural, formaldehyde, and glyoxal; methyl isobutyl ketone; toluene; protoanemonin; or acetone. Such impurities can be efficiently separated by purification or the like using the hydraulic wash column in the production method of the present invention. Thereby, a product can be efficiently obtained.

The hydraulic wash column used in the production method of the present invention may have any dimensions. Preferably, the inner diameter of the column (the crystallization chamber) is 30 to 2000 mm, for example. The height of the column is preferably 1000 to 15000 mm. Use of the production method of the present invention allows the crystal bed to be broken up substantially uniformly, even in an industrial-scale hydraulic wash column.

The circulating liquid feed unit in the hydraulic wash column may have any dimensions. Preferably, the inner diameter of the unit is 5 to 30 mm, for example. The height of the unit is preferably 10 to 300 mm.

The filter for filtering the crystal-containing slurry in the hydraulic wash column may have any dimensions. Preferably, the inner diameter of the filter is 10 to 30 mm, for example. The height of the filter is preferably 20 to 300 mm.

The filter may be provided with a large number of circular holes, slits (notches), or rectangular holes, for example. The filter may have the same shape as the pipe, such as a cylindrical shape, but is not limited thereto.

When the filter is provided with circular holes, the diameter of each hole may be appropriately adjusted depending on the size of the crystals, and is preferably 50 to 500 µm, for example. The number of holes is not limited, and may be adjusted according to the pressure loss, for example.

The pipe for discharging the mother liquor, which is connected to the filter, is usually disposed above the filter.

The number of pipes for discharging the mother liquor connected to the filter is not limited. For example, in an industrial-scale hydraulic wash column, one filter is installed per 30 to 90 cm² cross-sectional area of the hydraulic wash column and one pipe is connected to that one filter.

In the production method of the present invention, a mechanism for discharging crystals from the crystal bed formed in the hydraulic wash column, such as a rotor blade or a scraper, may further be used. Preferably, such a mechanism is not used. In particular, when the compound is an easily polymerizable compound having a reactive double bond, such a mechanism is more preferably not used. The reason for this is as follows. As described above, in the production method of the present invention, the compound is preferably an easily polymerizable compound having a reactive double bond, such as (meth)acrylic acid. Such a compound generates a polymer at a sealed part due to sliding heat when a scraper is used, which may cause leakage.

Preferably, the production method of the present invention further includes a step of heating an outer wall surface of the hydraulic wash column.

This makes it possible to prevent freezing and ensure stable use of the purification apparatus.

In the step of heating an outer wall surface of the hydraulic wash column, any heating unit may be used, and a heating medium, a steam tracing system, an electric tracing system, or a known heating unit that adjusts the environmental temperature of the column may be used. For example, part of the hydraulic wash column may be heated with a heating medium or the like, or substantially the entire hydraulic wash column may be heated (jacket heating).

The number of pipes that feed the crystal-containing slurry to the hydraulic wash column and the number of feed nozzles (slurry feed ports) that may be connected to the tips of the pipes are not limited. Each of the numbers may be one or more. (FIG. 1 shows the case where the number of pipes that feed the crystal-containing slurry to the hydraulic wash column is one).

The feed nozzle may have, at its tip, a distribution mechanism that distributes the slurry.

The hydraulic wash column may further include a distribution chamber and a central displacer body (see JP 2005-509010 T).

In the production method of the present invention, a dummy pipe that is connected to the filter for filtering the crystal-containing slurry in the hydraulic wash column may be used.

The dummy pipe is usually disposed below the filter.

The circulating liquid feed unit is preferably disposed below the dummy pipe, particularly preferably disposed directly below the dummy pipe.

The body or the periphery of the hydraulic wash column may be provided with instrumentation equipment such as a thermometer (e.g., a multi-point thermometer), a pressure gauge, or an interface level meter (e.g., an optical interface level meter).

The hydraulic wash column itself may be placed in a temperature-controlled casing (including a large casing such as a building).

### (Method of using hydraulic wash column)

The present invention also relates to a method of using a hydraulic wash column, the method including a step of purifying a compound using the hydraulic wash column in the present invention.

### EXAMPLES

The present invention will be described in more detail below with reference to examples, but the present invention is not limited by the following examples, and appropriate modifications may be made within the scope that can conform to the gist of the above and later descriptions. All of them are included in the technical scope of the present invention.

Unless otherwise specified, "%" indicates "% by mass" and "parts" indicates "parts by mass".

### (Example 1)

Propylene was subjected to catalytic gas-phase oxidation to obtain an acrylic acid-containing gas according to the method described in WO 2010/032665. Thereafter, the resulting acrylic acid-containing gas was introduced into an absorption column and collected with water to obtain an aqueous acrylic acid solution.

The aqueous acrylic acid solution was then fed to a crystallizer. The crystallizer is equipped with a jacket, and has a structure in which the contents in the crystallizer can be indirectly cooled by feeding a refrigerant to the jacket. The aqueous acrylic acid solution was cooled in the crystallizer and adhered to the inner surface of the crystallizer as crystals. These were scraped off with a scraper provided inside the crystallizer to obtain a crystal-containing slurry (feed slurry).

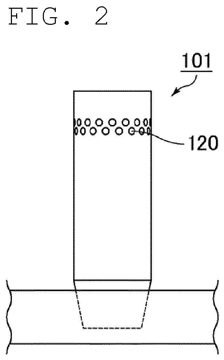

As a purification apparatus for obtaining a product from the crystal-containing slurry, the purification apparatus shown in FIGS. 1 and 2 was prepared. The specifications of the purification apparatus are as follows.
Hydraulic wash column 1: the number of slurry discharge ports 20 is 4
Filter 2: a filter made of PEEK and having circular pores with a diameter of 250 um
Mother liquor discharge pipe 3: a pipe made of stainless steel
Melt loop line (slurry discharge port 20, slurry circulation line 21, product discharge line 23, return line 24, and return port 25): the product discharge line 23 is equipped with a flow control valve (not shown)
Melting unit 22: heat exchanger
Entire apparatus: jacket structure (not shown)
Circulating liquid feed unit: the number of openings is 12 per unit (all horizontal ejection difference angles are approximately the same as each other), the number of rows of the openings is 1, the number density of the units is 45 cm²/unit

Using the above-mentioned purification apparatus, acrylic acid was purified from the crystal-containing slurry (feed slurry).

An acrylic acid crystal-containing slurry 11a was fed to the hydraulic wash column 1 through the slurry feed pipe 4 at a slurry concentration (acrylic acid crystal concentration) of 10% by mass, a slurry temperature of 9.3°C, and a feed amount of 4.63 t/h. The internal operating pressure of the hydraulic wash column 1 was set to 0.4 MPa, and a heat medium was introduced into a jacket (not shown) so that the temperature at the heat medium inlet was 25°C.

The circulating liquid feed units shown in FIG. 2 were disposed at approximately equal intervals at the bottom of the hydraulic wash column 1. In Example 1, the amount of the liquid ejected from one opening (one round hole) of each circulating liquid feed unit was 36 kg/h/hole, the amount of the liquid ejected from all the circulating liquid feed units was 3.0 t/h, and the melt was fed to the hydraulic wash column 1 at an ejection velocity of 3 m/s.

Next, the acrylic acid crystal-containing slurry was discharged at a linear velocity of 1.2 m/s from the slurry discharge ports 20 (four ports) provided at the bottom of the hydraulic wash column 1, and sent to the melting unit 22 (heat exchanger) at a flow rate of 3.23 t/h. In addition, the acrylic acid crystal-containing slurry was melted in the heat exchanger while controlling the temperature of the heat medium so that the liquid temperature at the outlet of the heat exchanger was 15°C. Thereby, a melt was prepared.

Part of the melt was removed as a product from the product discharge line 23 at a flow rate of 0.23 t/h. The remainder was returned to the hydraulic wash column 1 from the return port 25 via the return line 24.

The above operations allowed the crystal bed formed in the hydraulic wash column 1 to be broken up substantially uniformly, producing a product.

### (Example 2)

The same operations as in Example 1 were performed, except that the number of openings of each circulating liquid feed unit was set to 20 per unit, the number of rows of the openings was set to two (see FIG. 2), the amount of the liquid ejected from all the circulating liquid feed units was set to 5.0 t/h (the flow rate when the liquid was sent to the melting unit 22 was 5.23 t/h), and the linear velocity when the slurry was discharged from the slurry discharge ports 20 of the hydraulic wash column 1 was set to 2.0 m/s.

As a result, the crystal bed formed in the hydraulic wash column 1 was broken up substantially uniformly, producing a product.

### (Comparative Example 1)

The same operations as in Example 1 were performed, except that the number of openings of each circulating liquid feed unit was set to 8 per unit, the ejection velocity from each circulating liquid feed unit was set to 6 m/s, the amount of the liquid ejected from one opening (one round hole) of each circulating liquid feed unit was set to 71 kg/h/hole, the amount of the liquid ejected from all the circulating liquid feed units was set to 4.0 t/h (the flow rate when the liquid was sent to the melting unit 22 was 4.23 t/h), and the linear velocity when the slurry was discharged from the slurry discharge ports 20 of the hydraulic wash column 1 was set to 1.6 m/s.

As a result, the crystal bed formed in the hydraulic wash column 1 was broken up, but was not broken up substantially uniformly because the number of ejection holes was insufficient. Thereby, a product of the required quality was not obtained.

### (Comparative Example 2)

The same operations as in Example 1 were performed, except that the number of openings of each circulating liquid feed unit was set to 12 per unit, the number of rows of the openings was set to two (see FIG. 2), the ejection velocity from each circulating liquid feed unit was set to 1.5 m/s, the amount of the liquid ejected from one opening (one round hole) of each circulating liquid feed unit was set to 18 kg/h/hole, the amount of the liquid ejected from all the circulating liquid feed units was set to 1.5 t/h (the flow rate when the liquid was sent to the melting unit 22 was 1.73 t/h), and the linear velocity when the slurry was discharged from the slurry discharge ports 20 of the hydraulic wash column 1 was set to 0.6 m/s.

As a result, the crystal bed formed in the hydraulic wash column 1 was broken up, but was not broken up substantially uniformly because the ejection velocity was insufficient and the expansion angle was insufficient. Thereby, a product of the required quality was not obtained.

### (Reference Example 1)

The same operations as in Example 1 were performed, except that the number of openings of each circulating liquid feed unit was set to 20 per unit, the number of rows of the openings was set to two (see FIG. 2), the ejection velocity from each circulating liquid feed unit was set to 1.5 m/s, the amount of the liquid ejected from one opening (one round hole) of each circulating liquid feed unit was set to 4 kg/h/hole, the amount of the liquid ejected from all the circulating liquid feed units was set to 0.62 t/h (the flow rate when the liquid was sent to the melting unit 22 was 0.85 t/h), and the linear velocity when the slurry was discharged from the slurry discharge ports 20 of the hydraulic wash column 1 was set to 0.3 m/s.

As a result, adjusting the slurry feed amount and/or the slurry concentration allowed the crystal bed to be broken up substantially uniformly, producing a high-quality product.

### (Comparative Example 3)

The same operations as in Example 1 were performed, except that the number of openings of each circulating liquid feed unit was set to 8 per unit, the ejection velocity from each circulating liquid feed unit was set to 1.5 m/s, the amount of the liquid ejected from one opening (one round hole) of each circulating liquid feed unit was set to 4 kg/h/hole, the amount of the liquid ejected from all the circulating liquid feed units was set to 0.25 t/h (the flow rate when the liquid was sent to the melting unit 22 was 0.48 t/h), and the linear velocity when the slurry was discharged from the slurry discharge ports 20 of the hydraulic wash column 1 was set to 0.1 m/s.

As a result, the crystal bed formed in the hydraulic wash column 1 was not broken up, and the purification apparatus did not operate.

### (Example 3)

The same operations as in Example 2 were performed, except that the slurry feed amount was set to 10.4 t/h, the number density of the circulating liquid feed units was set to 101 cm²/unit, the ejection velocity from each circulating liquid feed unit was set to 6 m/s, the amount of the liquid ejected from one opening (one round hole) of each circulating liquid feed unit was set to 71 kg/h/hole, the amount of the liquid ejected from all the circulating liquid feed units was set to 10.0 t/h, the amount of a product to be produced was set to 0.52 t/h, and the linear velocity when the slurry was discharged from the slurry discharge ports 20 of the hydraulic wash column 1 was set to 1.4 m/s.

As a result, the crystal bed formed in the hydraulic wash column 1 was broken up substantially uniformly, producing a product. Still, since the number density of the circulating liquid feed units was rather sparse at 101 cm²/unit, the ejection velocity was adjusted to increase the coverage area of the ejected liquid.

### (Example 4)

The same operations as in Example 2 were performed, except that the slurry feed amount was set to 1.16 t/h, the number density of the circulating liquid feed units was set to 11 cm²/unit, the ejection velocity from each circulating liquid feed unit was set to 2 m/s, the amount of the liquid ejected from one opening (one round hole) of each circulating liquid feed unit was set to 24 kg/h/hole, the amount of the liquid ejected from all the circulating liquid feed units was set to 3.3 t/h, the amount of a product to be produced was set to 0.06 t/h, and the linear velocity when the slurry was discharged from the slurry discharge ports 20 of the hydraulic wash column 1 was set to 0.7 m/s.

As a result, the crystal bed formed in the hydraulic wash column 1 was broken up substantially uniformly, producing a product. Still, since the number density of the circulating liquid feed units was rather dense at 11 cm²/unit, the ejection velocity was adjusted to further avoid interference of the ejected liquid.

### (Example 5)

The same operations as in Example 2 were performed, except that the ejection velocity from each circulating liquid feed unit was set to 2 m/s, the amount of the liquid ejected from one opening (one round hole) of each circulating liquid feed unit was set to 24 kg/h/hole, the amount of the liquid ejected from all the circulating liquid feed units was set to 3.3 t/h (the flow rate when the liquid was sent to the melting unit 22 was 3.53 t/h), the linear velocity when the slurry was discharged from the slurry discharge ports 20 of the hydraulic wash column 1 was set to 1.3 m/s, and the liquid temperature at the outlet of the heat exchanger was set to 20°C.

As a result, the crystal bed formed in the hydraulic wash column 1 was broken up substantially uniformly, producing a product.

### (Example 6)

The same operations as in Example 2 were performed, except that the ejection velocity from each circulating liquid feed unit was set to 3.5 m/s, the amount of the liquid ejected from one opening (one round hole) of each circulating liquid feed unit was set to 42 kg/h/hole, the amount of the liquid ejected from all the circulating liquid feed units was set to 5.8 t/h (the flow rate when the liquid was sent to the melting unit 22 was 6.03 t/h), the linear velocity when the slurry was discharged from the slurry discharge ports 20 of the hydraulic wash column 1 was set to 2.3 m/s, and the liquid temperature at the outlet of the heat exchanger was set to 14°C.

As a result, the crystal bed formed in the hydraulic wash column 1 was broken up substantially uniformly, producing a product.

The results obtained above are summarized in Table 1 below.

The evaluation shown in Table 1 below was based on the following criteria.
∘: The crystal bed was broken up substantially uniformly, producing a high-quality product.
Δ: Adjusting the ejection velocity allowed the crystal bed to be broken up substantially uniformly, producing a high-quality product.
□: Adjusting the slurry feed amount and/or the slurry concentration allowed the crystal bed to be broken up substantially uniformly, producing a high-quality product.
×: The crystal bed was broken up, but was not broken up substantially uniformly, whereby a product of the required quality was not obtained.
×××: The crystal bed formed in the hydraulic wash column was not broken up, and the purification apparatus did not operate. Moreover, the circulating liquid feed units did not feed the liquid substantially uniformly around them.

The symbols "o", "Δ", and "□" indicate that the effects of the present invention are achieved.

In Table 1 below, the expansion angle was determined by measuring the expansion angle of water ejected in water at the same or almost the same velocity as the liquid ejected from a liquid ejection hole that has substantially the same peripheral thickness, shape, and size as any of the openings of the circulating liquid feed unit.

**[Table 1]**

| | | Example 1 | Example 2 | Com parative Example 1 | Com parative Example 2 | Reference Example 1 | Com parative Example 3 | Example 3 | Example 4 | Example 5 | Example 6 |
|---|---|---|---|---|---|---|---|---|---|---|---|
| Circulating liquid feed unit | | | | | | | | | | | |
| Number of openings | [pieces/unit] | 12 | 20 | 8 | 12 | 20 | 8 | 20 | 20 | 20 | 20 |
| Ejection velocity | [m/s] | 3 | 3 | 6 | 1.5 | 1.5 | 1.5 | 6 | 2 | 2 | 3.5 |
| Amount of liquid ejected (per hole) | [kg/hr/hole] | 36 | 36 | 71 | 18 | 4 | 4 | 71 | 24 | 24 | 42 |
| Amount of liquid ejected (total) | [t/hr] | 3.0 | 5.0 | 4.0 | 1.5 | 0.62 | 0.25 | 10.0 | 3.3 | 3.3 | 5.8 |
| Number of rows of provided openings | [Colum n] | 1 | 2 | 1 | 2 | 2 | 1 | 2 | 2 | 2 | 2 |
| Number density | [cm²/unit] | 45 | 45 | 45 | 45 | 45 | 45 | 101 | 11 | 45 | 45 |
| Tem perature of ejected liquid | [°C] | 15.0 | 15.0 | 15.0 | 15.0 | 15.0 | 15.0 | 15.0 | 15.0 | 20.0 | 14.0 |

| Hydraulic wash column | | | | | | | | | | | |
|---|---|---|---|---|---|---|---|---|---|---|---|
| Number of discharge ports | [pieces] | 4 | 4 | 4 | 4 | 4 | 4 | 4 | 4 | 4 | 4 |
| Linear velocity at discharge port | [m/s] | 1.2 | 2.0 | 1.6 | 0.6 | 0.3 | 0.1 | 1.4 | 0.7 | 1.3 | 2.3 |
| Slurry feed amount | [t/hr] | 4.63 | 4.63 | 4.63 | 4.63 | 4.63 | 4.63 | 10.4 | 1.16 | 4.63 | 4.63 |
| Slurry concentration | [% by mass] | 10 | 10 | 10 | 10 | 10 | 10 | 10 | 10 | 10 | 10 |
| Slurry temperature | [°C] | 9.3 | 9.3 | 9.3 | 9.3 | 9.3 | 9.3 | 9.3 | 9.3 | 9.3 | 9.3 |
| Amount of product | [t/hr] | 0.23 | 0.23 | 0.23 | 0.23 | 0.23 | 0.23 | 0.52 | 0.06 | 0.23 | 0.23 |
| Expansion angle | [°] | 30 | 30 | 31 | 21 | 21 | 21 | 31 | 23 | 23 | 30 |
| Evaluation | | ○ | ○ | × | × | □ | XXX | △ | △ | ○ | ○ |

The above results demonstrated that the crystal bed can be broken up substantially uniformly, thereby producing a high-quality product, using a hydraulic wash column including therein at least one circulating liquid feed unit including circulating liquid ejection holes for ejecting a crystal grain melt-containing circulating liquid into the hydraulic wash column, the circulating liquid feed unit including: a circulating liquid ejection part including the circulating liquid ejection holes in a number of M or more, M being calculated according to a specific requirement; and a circulating liquid feed area for feeding a circulating liquid from outside the hydraulic wash column into the circulating liquid ejection part, the circulating liquid being ejected from the circulating liquid ejection part into the hydraulic wash column through the circulating liquid ejection holes in a plurality of different substantially horizontal directions at a circulating liquid ejection velocity of 0.3 m/s or higher, with an amount of the liquid ejected from one circulating liquid ejection hole being 1.5 kg/h or more.

If the amount of the liquid ejected is less than 1.5 kg/h, the crystal bed may be difficult to be broken up. Still, specifying the number of ejection holes and the ejection velocity as described above allows the crystals to be handled substantially uniformly.

### REFERENCE SIGNS LIST

1: hydraulic wash column
2: filter
3: mother liquor discharge pipe
4: slurry feed pipe
11: slurry feed line
11a: crystal-containing slurry
11b: mother liquor
20: slurry discharge port
21: slurry circulation line
22: melting unit
23: product discharge line
23a: product
24: return line
25: return port
P: liquid delivery pump
101: circulating liquid feed unit
120: opening (circulating liquid ejection hole)

## Claims

1. A method comprising:
feeding a crystal grain melt-containing liquid into a vessel; and
discharging a crystal grain-containing slurry from the vessel,
the vessel including therein at least one liquid feed unit including liquid ejection holes for ejecting the melt-containing liquid into the vessel,
the liquid feed unit including:
a liquid ejection part including the liquid ejection holes in a number of M or more, M being calculated according to the following requirement; and
a liquid feed area for feeding the liquid from outside the vessel into the liquid ejection part,
the liquid being ejected from the liquid ejection part into the vessel through the liquid ejection holes in a plurality of different substantially horizontal directions at a liquid ejection velocity of 2 m/s or higher,
the requirement being as follows:
Requirement: M is a value obtained by dividing 360 by either of the following values and rounded down to the nearest whole number:
1) an expansion angle (°) of water ejected in water from a single liquid ejection hole equivalent to any of the liquid ejection holes at the same or almost the same velocity; and
2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

2. The method according to claim 1,
wherein in the at least one liquid feed unit, when viewed from the top to bottom direction, all horizontal ejection difference angles are substantially the same as each other, wherein each horizontal ejection difference angle denotes an angle formed by ejection directions of the liquid ejected from adjacent liquid ejection holes among the liquid ejection holes.

3. The method according to claim 1 or 2,
wherein the liquid ejection holes included in the liquid ejection part are provided on a circle having a center in the liquid ejection part when viewed from the top to bottom direction.

4. The method according to any one of claims 1 to 3,
wherein in the at least one liquid feed unit, the liquid ejection holes are provided at two or more different heights when viewed from the horizontal direction.

5. The method according to any one of claims 1 to 4,
wherein the slurry is discharged in at least four directions from the vessel at a linear velocity of 1 m/s or higher.

6. A method for producing a compound, the method comprising using a hydraulic wash column,
the hydraulic wash column including therein at least one circulating liquid feed unit including circulating liquid ejection holes for ejecting a crystal grain melt-containing circulating liquid into the hydraulic wash column,
the circulating liquid feed unit including:
a circulating liquid ejection part including the circulating liquid ejection holes in a number of M or more, M being calculated according to the following requirement; and
a circulating liquid feed area for feeding a circulating liquid from outside the hydraulic wash column into the circulating liquid ejection part,
the circulating liquid being ejected from the circulating liquid ejection part into the hydraulic wash column through the circulating liquid ejection holes in a plurality of different substantially horizontal directions at a circulating liquid ejection velocity of 2 m/s or higher, with an amount of the liquid ejected from one circulating liquid ejection hole being 10 kg/h or more.
the requirement being as follows:
Requirement: M is a value obtained by dividing 360 by either of the following values and rounded down to the nearest whole number:
1) an expansion angle (°) of water ejected in water from a single liquid ejection hole equivalent to any of the liquid ejection holes at the same or almost the same velocity; and
2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

7. The method according to claim 6,
wherein, in an inner view of the hydraulic wash column from the top to bottom direction, one circulating liquid feed unit is present per 30 to 90 cm².

8. The method according to claim 6 or 7,
wherein a temperature of the circulating liquid is higher than a melting point of the compound by not less than + 1°C.

9. The method according to any one of claims 6 to 8,
wherein the compound is an organic compound having a solid density higher than a liquid density of the organic compound.

10. The method according to any one of claims 6 to 9,
wherein the compound is (meth)acrylic acid.

11. A method comprising:
feeding a crystal grain melt-containing liquid into a vessel; and
discharging a crystal grain-containing slurry from the vessel,
the vessel including therein at least one liquid feed unit including liquid ejection holes for ejecting the melt-containing liquid into the vessel,
the liquid feed unit including:
a liquid ejection part including the liquid ejection holes in a number of M or more, M being calculated according to the following requirement; and
a liquid feed area for feeding the liquid from outside the vessel into the liquid ejection part,
the liquid being ejected from the liquid ejection part into the vessel through the liquid ejection holes in a plurality of different substantially horizontal directions at a liquid ejection velocity of 0.3 m/s or higher,
the requirement being as follows:
Requirement: M is a value obtained by dividing 360 by either of the following values and rounded down to the nearest whole number:
1) an expansion angle (°) of water ejected in water from a single liquid ejection hole equivalent to any of the liquid ejection holes at the same or almost the same velocity; and
2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.

12. A method for producing a compound, the method comprising using a hydraulic wash column,
the hydraulic wash column including therein at least one circulating liquid feed unit including circulating liquid ejection holes for ejecting a crystal grain melt-containing circulating liquid into the hydraulic wash column,
the circulating liquid feed unit including:
a circulating liquid ejection part including the circulating liquid ejection holes in a number of M or more, M being calculated according to the following requirement; and
a circulating liquid feed area for feeding a circulating liquid from outside the hydraulic wash column into the circulating liquid ejection part,
the circulating liquid being ejected from the circulating liquid ejection part into the hydraulic wash column through the circulating liquid ejection holes in a plurality of different substantially horizontal directions at a circulating liquid ejection velocity of 0.3 m/s or higher, with an amount of the liquid ejected from one circulating liquid ejection hole being 1.5 kg/h or more,
the requirement being as follows:
Requirement: M is a value obtained by dividing 360 by either of the following values and rounded down to the nearest whole number:
1) an expansion angle (°) of water ejected in water from a single liquid ejection hole equivalent to any of the liquid ejection holes at the same or almost the same velocity; and
2) an expansion angle (°) of liquid ejected from a single liquid ejection hole equivalent to any of the liquid ejection holes at the same or almost the same velocity as determined by flow analysis (numerical calculation), the liquid having the same or almost the same physical properties as the aforementioned liquid.
